# DEMANDE DE BREVET EUROPEEN

(11) **EP 1 621 530 A1**
(43) Date de publication de la demande: **01.02.2006**
(21) Numéro de dépôt: 05020919.6
(22) Date de dépôt: 06.12.2000
(51) Int. Cl.: C07C 237/20, C12Q 1/37, C12Q 1/04

(54) **Procédés, compositions et substrats enzymatiques pour la détection de pseudomonas aeruginosa**

(30) Priorité: 06.12.1999 FR 9915312
(62) Demande divisionnaire de: 00985413.4
(71) Demandeur: BIOMERIEUX SA, 69280 Marcy L'Etoile (FR)
(72) Inventeur: Desmonceaux, Mireille, 01150 Lagnieu (FR); Monget, Daniel, 01150 Saint-Sorlin-en-Bugey (FR)
(74) Mandataire: Anderson, Angela Mary

(57) **Abrégé**

La présente invention concerne un substrat enzymatique constitué d'une partie cible et d'une partie marqueur, l'hydrolyse du substrat permettant la séparation de la partie cible et de la partie marqueur, ladite partie cible caractérisant l'activité enzymatique recherchée. Elle concerne également une composition contenant au moins un tel substrat, ainsi qu'un procédé de détection de l'espèce bactérienne *Pseudomonas aeruginosa*.

La partie cible du substrat permet de révéler une activité enzymatique β-alanine aminopeptidase et la partie marqueur est une molécule révélant la réaction d'hydrolyse.

L'invention trouve une application préférentielle dans le domaine du diagnostic.

## Description

La présente invention concerne un substrat utilisable dans un test de détection de l'activité enzymatique β-alanine aminopeptidase. Elle concerne également une composition contenant au moins un tel substrat, ainsi qu'un procédé de détection de l'espèce bactérienne *Pseudomonas aeruginosa.*

Depuis de très nombreuses années, on utilise des substrats particuliers pour permettre la détermination de la présence ou de l'absence d'activités enzymatiques caractéristiques de micro-organismes. Par le choix des substrats, selon qu'il y a réaction ou non, il est possible de caractériser la nature d'un genre de micro-organismes ou de différencier des souches et/ou des espèces d'un genre microbien donné.

Les substrats synthétiques d'enzymes sont constituées de deux parties, une première partie spécifique de l'activité enzymatique à révéler, ci-après appelée partie cible, et une seconde partie faisant office de marqueur, ci-après appelée partie marqueur.

Ces substrats particuliers peuvent être fluorescents ou chromogènes. En fait, c'est la seconde partie marqueur ou le produit de sa réaction avec un ou plusieurs autres composés, voir à ce propos la demande de brevet PCT/FR99/00781 déposée au nom de la demanderesse, qui est fluorescente ou chromogène lorsqu'elle n'est plus associée à la première partie cible.

Or l'état de la technique ne décrit pas de test simple pour caractériser l'espèce *Pseudomonas aeruginosa,* qui reste pourtant une espèce des plus recherchées du fait de sa grande pathogénicité, sa fréquence de distribution assez importante et son implication dans de nombreuses maladies nosocomiales.

Conformément à la présente invention, on propose une gamme de substrats à base de β-Alanine comme partie cible, qui permettent tous une réelle détection de *Pseudomonas aeruginosa.* L'invention concerne encore une composition contenant au moins un tel substrat ainsi qu'un précédé de détection de *Pseudomonas aeruginosa.*

Certes l'activité β-Alanine est déjà bien connue. Ainsi dans un article de Kasafirek, Evzen et al., intitulé « Rôle of amino acid residues in chromogenic substrates cleaved by pancreatic elactase », Collect. Czech. Chem. Commun. (1987), 52(6), 1625-33, il est mentionné des substrats chromogéniques comme par exemple la β-Alanine p-Nitroaniline (voir sa synthèse en page 1631).

La différence, qui nous semble essentielle par rapport à la présente invention, a pour objet la cible que nous visons à savoir *Pseudomonas aeruginosa,* et non l'enzyme d'Elastase pancréatique.

Un article provenant de J.B. Suszkiw, A. S. Brecher, sur « Brain Aminoacyl Arylamidase. Further Purification of the Soluble Bovine Enzyme and Studies on Substrate Specificity and Possible Active-Site Residues », Biochemistry, vol. 9, no. 20 (1970), pages 4008-4017, propose une technique de purification d'une Arylamidase soluble extraite de l'encéphale de bovins, la β-Alanine β-naphtylamine (voir sa synthèse en page 4010 2^{ème} alinéa).

Là encore, la cible est complètement différente de celle que nous envisageons.

A cet effet, la présente invention concerne un substrat enzymatique constitué d'une partie cible et d'une partie marqueur, l'hydrolyse du substrat permettant la séparation de la partie cible et de la partie marqueur, ladite partie cible caractérisant l'activité enzymatique recherchée, caractérisé par le fait que la partie cible permet de révéler une activité enzymatique β-alanine aminopeptidase d'au moins un micro-organisme *Pseudomonas aeruginosa,* et la partie marqueur est une molécule révélant la réaction d'hydrolyse.

Selon une variante préférentielle de réalisation, la partie cible est constituée de β-Alanine ou d'un de ses dérivés et la partie marqueur est une molécule chromogène ou fluorescent.

Toujours selon une variante préférentielle de réalisation, le substrat est de formule :

H₂N-CH₂-CH₂-CO-NH-R,

où H₂N-CH₂-CH₂-CO- est la partie cible et -NH-R est la partie marqueur chromogène ou fluorescent du substrat.

Encore selon une variante préférentielle de réalisation, la partie marqueur est constituée de :
- β-Naphtylamine,
- Aminométhylcoumarine, tel que 7-amino-4-méthyl-coumarine,
- dérivé de l'Aminobenzène, tel que 4-amino-2,6-dichlorophénol,
- p-Nitroaniline, ou
- 2-amino-indoxyl ou 3-amino-indoxyl.

L'invention concerne également une composition permettant la détection d'au moins une souche et/ou une espèce de micro-organismes qui comprend au moins un substrat, tel que décrit ci-dessus, et un milieu de culture.

Selon une variante, la composition permettant la détection d'au moins souche et une espèce de micro-organismes ou d'au moins deux souches ou deux espèces de micro-organismes, qui comprend au moins un substrat, tel que décrit ci-dessus, au moins un autre substrat et un milieu de culture.

Selon une variante, le milieu de culture contient un révélateur.

Dans ce cas, le milieu de culture contient comme révélateur :
- du sel de diazonium, lorsque la partie marqueur libérée est la β-Naphtylamine, ou
- de l'Acide 3,5-dihydroxy-2-naphtoïque, lorsque la partie marqueur libérée est un dérivé de l'Aminobenzène.

Préférentiellement, la composition est sous la forme d'un milieu liquide ou d'un milieu gélosé.

L'invention concerne enfin un procédé de détection de l'espèce bactérienne *Pseudomonas aeruginosa,* ce procédé consiste :
- à mettre au moins un substrat permettant de détecter l'activité enzymatique β-alanine aminopeptidase en présence d'un échantillon suspecté de contenir au moins un micro-organisme *Pseudomonas aeruginosa,* et
- à observer l'apparition de réactions colorées et/ou fluorescentes.

Selon une variante, le procédé de détection de l'espèce bactérienne *Pseudomonas aeruginosa,* consiste :
- à mettre au moins un substrat permettant de détecter l'activité enzymatique β-alanine aminopeptidase en présence d'un échantillon suspecté de contenir au moins un micro-organisme *Pseudomonas aeruginosa*,
- à mettre au moins un substrat permettant de détecter une activité enzymatique différente de β-alanine aminopeptidase en présence de l'échantillon suspecté de contenir au moins un autre micro-organisme que *Pseudomonas aeruginosa,* cette autre activité enzymatique permettant de différencier *Pseudomonas aeruginosa* de cet autre micro-organisme, et
- à observer l'apparition de réactions colorées et/ou fluorescentes.
   Dans les deux cas de figure ci-dessus, le milieu de culture est gélifié.
   Dans ce cas, on ajoute dans ce milieu de culture un produit réduisant la migration de la ou des colorations obtenues.
   La présente invention concerne l'utilisation d'une propriété biologique propre à la plupart des souches de l'espèce bactérienne *Pseudomonas aeruginosa,* qui possède une activité β-alanine aminopeptidase. Cette propriété ne se retrouve que chez de rares autres espèces.
   L'invention consiste à associer à la partie cible spécifique de l'enzyme, qui est constituée par la β-Alanine, une partie marqueur adaptée. De telles parties marqueurs pour révéler cette activité existent déjà, mais leur utilisation pour détecter *Pseudomonas aeruginosa* n'est pas connue, puisque le lien entre cette espèce et cette activité enzymatique n'a jamais été décrite.
   Ainsi, la première famille de substrats est constituée d'une molécule ayant les Naphtylamines comme partie marqueur, dont l'une des formules, correspondant au substrat β-alanyl-β-naphtylamide, est :

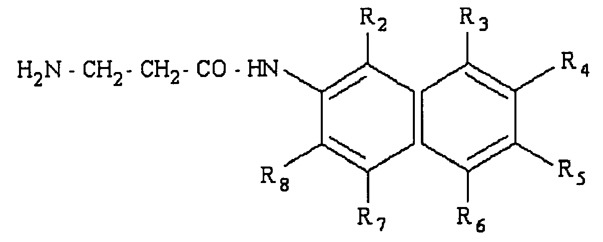

dans laquelle R₂, R₃, R₄, R₅, R₆, R₇, et R₈ représentent un atome d'hydrogène ou de brome ou de chlore ou d'iode ou par un groupement de type -OH, -CH₃, -CH₂CH₃, - OCH₃, -OCH₂CH₃ ou COOH à chacune de ces positions. La β-Naphtylamine peut être utilisée pour détecter l'activité β-alanine aminopeptidase, c'est ce que décrit la thèse de Docteur-Ingénieur de monsieur Daniel Monget, soutenue devant l'Université Claude Bernard - Lyon I le 4 juillet 1978 (N° d'ordre 297). Ce type de molécules étant incolore, il convient pour qu'elles soient utilisables d'ajouter un révélateur, tel que du sel de diazonium.

La deuxième famille de substrats de l'état de la technique est constituée par des molécules dont la partie marqueur est à base d'Aminométhylcoumarine, qui est fluorescente une fois libérée de sa partie cible. La formule générale de cette famille de substrats peut être déduite de la formule particulière suivante, qui correspond à la β-Alanyl-7-amido-4- méthylcoumarine : 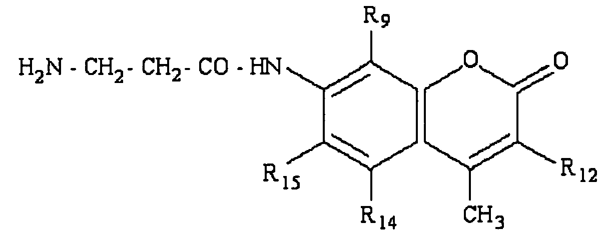
dans laquelle R₉, R₁₂, R₁₃, R₁₄ et R₁₅ représentent un atome d'hydrogène ou de brome ou de chlore ou d'iode ou par un groupement de type -OH, -CH₃, -CH₂CH₃, -OCH₃, -OCH₂CH₃ ou COOH à chacune de ces positions. Ce type de substrats est peu adapté à une utilisation dans des milieux gélosés, et est plus utilisé en milieu liquide.

La troisième famille de substrats est constituée de β-Alanyl-4-amino-2,6-dichlorophénol ayant le 2,6-Dichloroaminophénol comme partie marqueur, dont la formule globale est la suivante : 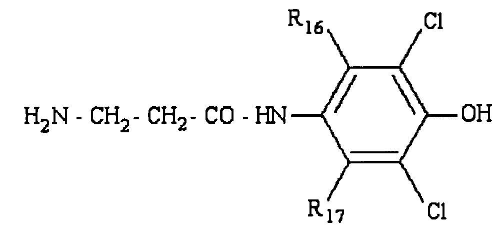
dans laquelle R₁₆ et R₁₇ représentent un atome d'hydrogène ou de brome ou de chlore ou d'iode ou par un groupement de type -OH, -CH₃, -CH₂CH₃, -OCH₃, -OCH₂CH₃ ou COOH à chacune de ces positions. Ce type de molécules a déjà été décrite dans une précédente demande de brevet WO-A-99/51767, déposée par la demanderesse, et nécessite la présence d'un révélateur séché, par exemple l'Acide 3,5 dihydroxy-2-naphtoïque. Toutefois, cette demande de brevet WO-A-99/51767 montre qu'il est possible d'utiliser d'autres molécules où les deux atomes de chlore et le groupement hydroxyle sont remplacés par un atome d'hydrogène ou de brome ou de chlore ou d'iode ou par un groupement de type -OH, -CH₃, -CH₂CH₃, -OCH₃, -OCH₂CH₃ ou COOH à chacune de ces positions.

La quatrième famille de substrats est à base de β-Alanyl p-nitroanilide ou dérivés, avec la p-Nitroaniline comme partie marqueur. La formule générale de ce type de substrats est la suivante : 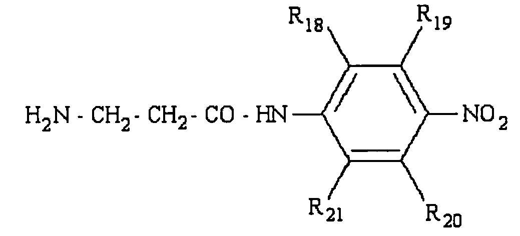
dans laquelle R₁₈, R₁₉, R₂₀ et R₂₁ représentent un atome d'hydrogène ou de brome ou de chlore ou d'iode ou par un groupement de type -OH, -CH₃, -CH₂CH₃, -OCH₃, -OCH₂CH₃ ou COOH à chacune de ces positions. Ce produit ne nécessite pas la présence d'un révélateur.

La cinquième famille de substrats est à base de 2-Amino-indole ou de 3-Amino-indole comme partie marqueur. La formule générale de ces substrats est la suivante pour le 2-Amino-indole: 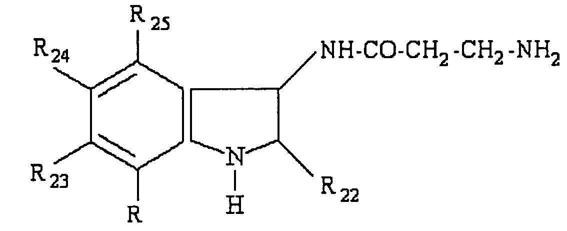 et pour le 3-Amino-indole :

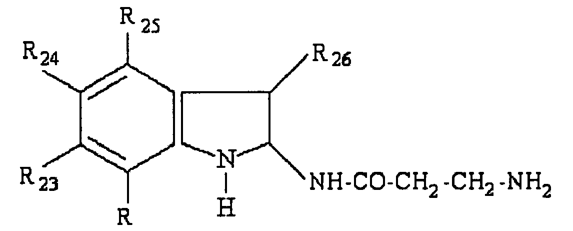

Tous ces substrats sont chromogènes à l'exception de la deuxième famille qui est fluorescente, et constituent tous des molécules permettant l'identification de l'activité enzymatique β-alanine aminopeptidase de *Pseudomonas aeruginosa.* Cette identification permet de bien différencier cette espèce pathogène en clinique d'autres espèces de bacilles à Gram négatif proches.

### 1°) Substrats utilisés :

Les substrats enzymatiques utilisés sont de formule :

H₂N-CH₂-CH₂-CO-NH-R,

où H₂N-CH₂-CH₂-COOH est la β-Alanine, c'est-à-dire la partie cible du substrat et -NH₂-R est le chromogène, c'est-à-dire la partie marqueur dudit substrat.

Quatre substrats ont été testés qui correspondent aux quatre premières familles de substrats précédemment décrites. Il y a :
- la β-Alanyl-β-naphtylamide, qui est commercialisée sous la référence K1035 par la société Bachem, Bubendorf, Suisse,
- la β-Alanyl-7-amido-4-méthylcoumarine, qui est commercialisée sous la référence I1030 par la société Bachem, Bubendorf, Suisse,
- la β-Alanyl-4-amino-2,6-dichlorophénol, dont la synthèse sera exposée ci-dessous, et
- la β-Alanine p-nitroanilide dont la synthèse sera exposée plus loin.

La synthèse de la β-Alanyl-4-amino-2,6-dichlorophénol est effectuée à partir de N-Tert-butoxycarboxyl-β-alanine (1,89 g, 10 mmol). Ce N-Tert-butoxycarboxyl-β-alanine est dissous dans du Tétrahydrofurane anhydre (HPLC grade, 30 ml). La solution est refroidie à 0°C en utilisant un bain de glace et de sel, et on ajoute du N-méthylmorpholine (2,02 g, 20 mmol). On porte la température en-dessous de -10°C, et on ajoute l'Isobutyl chloroformate (2,74g, 20 mmol) graduellement tout en conserver la température en-dessous de -8°C. On ajoute à la suspension anhydre ci-dessus, une solution froide de 4-Amino-2,6-dichlorophénol (1,78 g, 10mmol), dissous dans du Diméthylformamide et traitée avec du N-méthylmorpholine (4,08 g, 40 mmol) pour libérer la base libre. Cette solution est maintenue en-dessous de la température ambiante avant l'addition ci-dessus mentionnée. Le mélange réactionnel obtenu est agité dans un bain de glace et de sel pendant 1 heure, et ensuite pendant 5 heures à la température ambiante. Une chromatographie couche mince indique une conversion presque complète de produit en amide. La suspension est filtrée pour éliminer l'Hydrochlorure de N-méthylmorpholine, le résidu étant lavé en ajoutant du Tétrahydrofurane. Ensuite on utilise un vaporisateur rotatif pour retirer le Tétrahydrofurane et un peu de Diméthylformamide, et puis on verse lentement la solution obtenue dans de l'eau glacée vigoureusement agitée. Le précipité gris est repris, bien lavé à l'eau et séché dans un four à vide à 30-40°C. Le produit est recristallisé dans le méthanol avec l'addition d'un peu d'eau, ce qui donne 1,72 g de petits cristaux blancs. Il s'agit du N-α-t-BOC-β-alanyl-4-amino-2,6-dichlorophénol qui est ensuite dissous dans un minimum d'Acétate d'éthyle saturé de chlorure d'hydrogène. Après 2 heures à environ 16°C, la solution visqueuse est versée doucement dans 250 ml d'Ether diéthylique anhydre. Après 4 heures, le solide pulvérulent est récupéré par filtration sous vide et lavé à l'Ether diéthylique. Le produit est enfin séché dans un dessiccateur à vide et isolé pour minimiser l'hygroscopicité.

La synthèse de la β-Alanine p-nitroanilide est la suivante. Une masse de 2,76 grammes (g), soit 20 millimoles (mmol) de 4-Nitroaniline est dissoute par agitation en présence de 40 millilitres (ml) de pyridine séchée et préalablement refroidie à une température de moins 12°C. D'autre part, du Trichlorure de phosphore (1 ml redistillé) est ajouté à 12 ml de pyridine, toujours à la température de moins 12°C. Les deux solutions sont ensuite refroidies à moins 16°C et la deuxième solution contenant le Trichlorure de phosphore est ajoutée sous agitation à la première solution contenant la Nitroaniline. Ce mélange s'effectue entre moins 14 et moins 16°C pendant au moins 30 minutes (mn) puis à température ambiante pendant la même durée. On ajoute ensuite par petites quantités successives 20 mmol, soit 4,46 g, du Benzyloxycarbonyl-β-Alanine sec à la solution ci-dessus agitée pendant au moins 5 mn. Cette agitation se poursuit pendant 14 heures (h) entre 30 et 40°C. La température est ensuite portée à 50°C et l'agitation est pousuivie encore pendant 8 h. Une chromatographie en couche mince d'un échantillon dilué montre qu'une grande partie des produits de base a été transformée en dérivé β-aminoacyl. On retire ensuite la pyridine par évaporation rotative à 50°C. L'huile visqueuse résiduelle est agitée vigoureusement avec de la glace pilée en présence d'éthanol. On obtient ainsi en partie un solide jaunâtre sur les parois du récipient le contenant et en partie une masse cristalline. Le solide est repris, filtré par aspiration et lavé avec de l'eau. Le produit séché à l'air est recristallisé dans l'éthanol chaud. Le produit sec pèse 4,3 g et est homogène par chromatographie en couche mince (CCM) utilisant des plaques de gel de silice (solvant - éthyl acétate/toluène). Le rendement est de 62,7%. La protection constituée par le Benzyloxycarbonyl, présent dans la Benzyloxycarbonyl-β-alanine-p-nitroanilide, est ensuite éliminée. Ainsi, 2,15 g de cette substance sont dissous dans un volume minimal d'acide acétique glacial chaud sous agitation. A ce mélange, refroidi à 25°C est ajouté un volume égal de Bromure d'hydrogène à 30 % dans de l'acide acétique glacial sous agitation. Après 1 h 30 mn, la solution visqueuse est versée lentement dans 400 ml d'Ether diéthyl anhydre, sous agitation forte. La suspension blanche obtenue est ensuite filtrée et le résidu est lavé plusieurs fois par de l'éther anhydre, puis séché dans un dessiccateur à vide toute une nuit. On obtient ainsi 1,50 g de β-Alanine-p-nitroanilide, sous forme de sel de bromure.

### 2°) MISE EN EVIDENCE DE L'ACTIVITE ENZYMATIQUE β-ALANINE AMINOPEPTIDASE AU MOYEN DE DIFFERENTS SUBSTRATS ENZYMATIQUES:

### A - Test du substrat β-Alanyl-β-naphtylamide et de la sélectivité de la partie cible :

Plusieurs espèces, à raison de cinq souches par espèce, sauf si le nombre est précisé, ont été testées en présence de β-alanyl-β-naphtylamide dans des galeries de type API ZYM de chez bioMérieux La Balme, France. Les réactions ont été effectuées avec des suspensions bactériennes à une densité de 4 Mc Farland (McF) dans du tampon phosphate à 0,01M à pH 7,5. L'échelle de Mc Farland est une échelle de concentration bactérienne, où 4 McF correspondent à 12.10⁸ bactéries par millilitre. Pour chaque souche, 80 microlitres de suspension sont introduits dans chacune des cupules de la galerie. Les galeries sont mises à incuber à 37°C pendant 4 h. La présence β-alanine aminopeptidase est mise en évidence en ajoutant une goutte de ZYMB (sel de diazonium, Fast Blue BB), commercialisé par bioMérieux sous la référence 70480. L'apparition d'une coloration orange témoigne d'une réaction positive. Les résultats sont les suivants. Il y a une réaction négative pour les espèces énumérées ci-après, en d'autres termes aucune activité β-alanine aminopeptidase n'a été détectée :
- *Escherichia coli,*
- *Shigella* spp. (4 souches),
- *Shigella sonnei,*
- *Edwarsiella tarda* (2 souches),
- *Salmonella choleraesuis,*
- *Salmonella typhi*,
- *Salmonella* spp.,
- *Salmonella paratyphi A,*
- *Salmonella gallinarum* (3 souches),
- *Salmonella arizonae,*
- *Citrobacter freundii,*
- *Citrobacter diversus,*
- *Klebsiella pneumoniae,* spp. *pneumoniae,*
- *Klebsiella pneumoniae,* spp. *oxytoca,*
- *Klebsiella ozaenae,*
- *Klebsiella rhinoscleromatis* (3 souches),
- *Hafnia alvei,*
- *Enterobacter aerogenes,*
- *Enterobacter cloacae,*
- *Enterobacter sakazakii,*
- *Enterobacter gergoviae,*
- *Serratia marcescens,*
- *Serratia odorifera*,
- *Serratia fonticola*,
- *Serratia plymuthica*,
- *Proteus vulgaris,*
- *Proteus mirabilis,*
- *Proteus morganii,*
- *Proteus rettgeri,*
- *Providencia alcalifaciens,*
- *Providencia stuartii,*
- *Yersinia enterocolitica,*
- *Yersinia pseudotuberculosis*,
- *Pseudomonas putida,*
- *Comamonas acidovorans,*
- *Comamonas testosteroni,*
- *Pseudomonas alcaligenes,*
- *Pseudomonas stutzeri,*
- *Shewanella putrefaciens,*
- *Stenotrophomonas maltophilia,*
- *Brevundimonas diminuta,*
- *Brevundimonas vesicularis,*
- *Ralstonia picketti,*
- *Pseudomonas luteola,*
- *Pseudomonas oryzihabitans,*
- *Sphingomonas paucimobilis,*
- *Sphingobacterium multivorum,*
- *Alcaligenes faecalis*/*odorans*,
- *Achromobacter xylosoxidans* spp. *denitrificans,*
- *Achromobacter xylosoxidans,* spp. *xylosoxidans*,
- *Bordetella bronchiseptica,*
- *Oligella ureolytica* (2 souches),
- *CDC IV C*_{*2*}*,*
- *Aeromonas hydrophila*,
- *Plesiomonas shigelloïdes*,
- *Vibrio alginolyticus*,
- *Vibrio parahaemolyticus* (4 souches),
- *Chryseobacterium meningosepticum,*
- *Empedobacter brevis* (3 souches),
- *Chryseobacterium balustinum* (1 souche),
- *Chryseobacterium indologenes,*
- *Weeksella virosa* (4 souches),
- *Myroïdes* spp.,
- *Pasteurella gallinarum* (1 souche),
- *Pasteurella multocida,*
- *Pasteurella pneumotropica* (4 souches),
- *Actinobacillus ureae* (1 souche),
- *Pasteurella haemolytica*,
- *Pasteurella aerogenes*,
- proches de *Pasteurella* (4 souches),
- *Psychrobacter phenylpyruvicus* (4 souches),
- *Moraxella paraphenylpyruvica* (3 souches),
- *Moraxella lacunata* (4 souches),
- *Moraxella lac. sp. liquefaciens* (7 souches),
- *Moraxella non liquefaciens* (13 souches),
- *Moraxella bovis* (4 souches),
- *Moraxella osloensis* (18 souches), et
- *Actinobacillus* spp.

Par contre, il y a une réaction positive pour les quelques espèces énumérées ci-après, en d'autres termes une activité β-alanine aminopeptidase a été détectée :
- *Serratia liquefaciens,*
- *Pseudomonas aeruginosa,*
- *Pseudomonas fluorescens,*
- *Burkholderia cepacia,*
- *Pseudomonas mendocina.,* et
- *Ochrobactrum anthropi.*

CDC veut dire Center for Disease Control. Les groupes CDC sont des désignations de groupes de souches en attente d'un nom d'espèce.

Le substrat β-alanyl-β-naphtylamide et plus particulièrement la partie cible, la β-alanine, est donc particulièrement sélective puisque six (6) espèces seulement possèdent l'activité β-alanine aminopeptidase, pour soixante-dix-neuf (79) espèces qui ne la possèdent pas, soit environ 7,1 % d'espèces positives parmi celles testées.

La différenciation de *Pseudomonas aeruginosa* parmi les six espèces peut facilement être réalisée en milieu gélosé par la présence d'une coloration verte due à la production de pyoverdine par cette espèce.

### B - Test du substrat β-Alanyl-7-amido-4-méthylcoumarine et efficacité sur la détection de souches de Pseudomonas aeruginosa :

Le substrat β-Alanyl-7-amido-4-méthylcoumarine a été utilisé avec la méthodologie suivante. Les souches ont été isolées sur les milieux bioMérieux, France, suivants :
- Columbia sang de mouton,
- Trypticase soja sang de mouton, et
- Mac Conkey (milieu d'isolement des entérobactéries).

Les réactions ont été effectuées dans des cartes Vitek 2 (Marque déposée, bioMérieux, Saint Louis, Missouri, Etats-Unis d'Amérique) avec des suspensions bactériennes ajustées à une densité comprise entre 0,375 et 0,750 McF.

Le tableau 1 ci-dessous met en évidence l'intérêt ou non du substrat testé pour certaines espèces déjà abordées au chapitre précédent, ou pour de nouvelles espèces (par exemple *Ochrobactrum anthropi*).

Des tests simples comme l'hydrolyse de l'arginine (ADH) ou l'utilisation de l'acétamide permettent de séparer *Pseudomonas aeruginosa* de l'espèce *Ochrobactrum anthropi*.

| ESPECES | Nombre de souches testées | Nombre de réactions négatives | Nombre de réactions positives | Réactions difficiles à interpréter |
|---|---|---|---|---|
| *Shigella boydii* | 10 | 10 | 0 | 0 |
| *Shigella dysenteriae* | 10 | 10 | 0 | 0 |
| *Shigella flexneri* | 10 | 10 | 0 | 0 |
| *Chromobacterium violaceum* | 11 | 9 | 1 | 1 |
| *Chryseobacterium indologenes* | 10 | 10 | 0 | 0 |
| *Ochrobactrum anthropi* | 11 | 0 | 11 | 0 |
| *Oligella urethralis* | 10 | 10 | 0 | 0 |
| *Pseudomonas aeruginosa* | 137 | 2 | 134 | 1 |
| *Ralstonia pickettii* | 10 | 8 | 2 | 0 |
| *Pseudomonas mendocina* | 10 | 0 | 10 | 0 |
| *Pseudomonas stutzeri* | 10 | 10 | 0 | 0 |
| *Comamonas testoteroni* | 10 | 10 | 0 | 0 |

### Tableau 1 : Efficacité du substrat β-Alanyl-7-amido-4-méthylcoumarine

Le substrat est β-Alanyl-7-amido-4-méthylcoumarine est donc particulièrement efficace pour détecter les espèces :
- *Ochrobactrum anthropi,* puisque le taux de réactions positives est de 100%,
- *Pseudomonas aeruginosa,* puisque le taux de réactions positives est de 97,8%, et
- *Pseudomonas mendocina,* puisque le taux de réactions positives est de 100%.

La valeur trouvée pour *Pseudomonas aeruginosa* est particulièrement intéressante, car l'échantillonage testé comprend 137 espèces. Les trois souches, qui ne réagissent pas ou qui réagissent mal, sont liées à la variabilité biologique de cette espèce.

D'autres espèces montrent une aptitude partielle à hydrolyser la β-alanyl-7-amido-4- méthylcoumarine, il s'agit de :
- *Ralstonia pickettii,* puisque le taux de réactions positives est de 20%, et
- *Chromobacterium violaceum,* puisque le taux de réactions positives est de 10%.

### C - Test du substrat β-Alanyl-4-amino-2,6-dichlorophénol et efficacité sur la détection

### de souches de Pseudomonas aeruginosa :

Après isolement sur gélose Columbia au sang de mouton, les souches sont testées en milieux liquide sur des cartes Vitek 2 (Marque déposée, bioMérieux, Saint Louis, Missouri, Etats-Unis d'Amérique), contenant le substrat β-Alanyl-4-amino-2,6-dichlorophénol, en présence de l'Acide 3,5-dihydroxy-2-naphtoïque et un milieu réactionnel peptoné et tamponné à pH 8,5. Les densités des suspensions bactériennes sont de 0,5MF / NaCl 4,5 g/l. La lecture visuelle s'effectue après 22 à 24 heures d'incubation. Le tableau 2 ci-après expose les résultats qui ont été trouvés.

| ESPECES | Nombre de souches testées | Nombre de réactions négatives | Nombre de réactions positives |
|---|---|---|---|
| *Citrobacter amalonaticus* | 2 | 2 | 0 |
| *Citrobacter freundii* | 2 | 2 | 0 |
| *Citrobacter koseri* | 1 | 1 | 0 |
| *Enterobacter aerogenes* | 2 | 2 | 0 |
| *Enterobacter cloacae* | 2 | 2 | 0 |
| *Enterobacter intermedius* | 1 | 1 | 0 |
| *Escherichia coli* | 2 | 2 | 0 |
| *Proteus mirabilis* | 2 | 2 | 0 |
| *Proteus vulgaris* | 1 | 1 | 0 |
| *Providencia stuartii* | 1 | 1 | 0 |
| *Salmonella arizonae* | 1 | 1 | 0 |
| *Serratia liquefaciens* | 1 | 0 | 1 |
| *Serratia marcescens* | 3 | 0 | 3 |
| *Klebsiella oxytoca* | 2 | 2 | 0 |
| *Klebsiella pneumoniae* | 3 | 3 | 0 |
| *Achromobacter xylosoxydans* | 2 | 2 | 0 |
| *Acinetobacter baumannii* | 2 | 2 | 0 |
| *Aeromonas hydrophila* | 2 | 2 | 0 |
| *Brevundimonas diminuta* | 2 | 2 | 0 |
| *Burkholderia vesicularis* | 1 | 1 | 0 |
| *Chryseobacterium meningosepticum* | 2 | 2 | 0 |
| *Ochrobactrum anthropi* | 1 | 0 | 1 |
| *Moraxella non liquefaciens* | 2 | 2 | 0 |
| *Myroïdes* spp. | 1 | 1 | 0 |
| *Pseudomonas aeruginosa* | 13 | 0 | 13 |
| *Pseudomonas fluorescens* | 2 | 1 | 1 |
| *Pseudomonas mendocina* | 1 | 0 | 1 |
| *Pseudomonas putida* | 1 | 1 | 0 |
| *Pseudomonas stutzeri* | 3 | 3 | 0 |
| *Ralstonia pickettii* | 1 | 1 | 0 |
| *Shewanella algae* | 1 | 1 | 0 |
| *Shewanella putrefaciens* | 1 | 1 | 0 |
| *Sphingomonas paucimobilis* | 2 | 2 | 0 |
| *Stenotrophomonas maltophilia* | 2 | 2 | 0 |
| *Streptococcus agalactiae* | 1 | 1 | 0 |
| *Streptococcus pyogenes* | 2 | 2 | 0 |
| *Enterococcus faecalis* | 2 | 2 | 0 |
| *Enterococcus faecium* | 2 | 2 | 0 |
| *Vibrio vulnificus* | 1 | 1 | 0 |

### Tableau 2 : Efficacité du substrat β-Alanyl-4-amino-2,6-dichlorophénol

Avec ce substrat, β-Alanyl-4-amino-2,6-dichlorophénol, on obtient des résultats qui sont toujours très significatifs avec *Pseudomonas aeruginosa, Serratia liquefaciens, Serratia marcescens* et relativement significatif avec *Pseudomonas mendocina* et *Ochrobactrum anthropi*. Il peut donc y avoir un intérêt à combiner ce substrat avec un autre substrat permettant de distinguer *Pseudomonas aeruginosa* des autre micro-organismes qui ont une réactivité positive (voir 3^{ème} paragraphe). Le cas de *Pseudomonas fluorescens* est difficile à interpréter et relève plutôt d'un problème de manipulation ou de la variabilité génétique de cette espèce.

De plus, on peut noter que la présence de polyvinyl pyruvate (PVP) améliore la coloration des réactions positives.

### D - Test du substrat β-Alanyl para-nitroanilide :

Il s'agit sensiblement de la même méthodologie que dans le paragraphe C précédent, à l'exception de la lecture visuelle qui s'effectue après 19h d'incubation. On a utilisé comme substrat la β-Alanyl p-nitroanilide. Le tableau 3 ci-après récapitule les résultats.

| ESPECES | Nombre de souches testées | Nombre de réactions négatives | Nombre de réactions positives |
|---|---|---|---|
| *Citrobacter amalonaticus* | 2 | 2 | 0 |
| *Citrobacter freundii* | 2 | 2 | 0 |
| *Citrobacter koseri* | 1 | 1 | 0 |
| *Enterobacter aerogenes* | 2 | 2 | 0 |
| *Enterobacter cloacae* | 2 | 2 | 0 |
| *Enterobacter intermedius* | 1 | 1 | 0 |
| *Escherichia coli* | 2 | 2 | 0 |
| *Proteus mirabilis* | 2 | 2 | 0 |
| *Proteus vulgaris* | 1 | 1 | 0 |
| *Providencia stuartii* | 1 | 1 | 0 |
| *Salmonella arizonae* | 1 | 1 | 0 |
| *Serratia liquefaciens* | 1 | 0 | 1 |
| *Serratia marcescens* | 1 | 0 | 1 |
| *Klebsiella oxytoca* | 2 | 2 | 0 |
| *Klebsiella pneumoniae* | 2 | 2 | 0 |
| *Achromobacter xylosoxydans* | 2 | 2 | 0 |
| *Acinetobacter baumannii* | 2 | 2 | 0 |
| *Aeromonas hydrophila* | 2 | 2 | 0 |
| *Brevundimonas diminuta* | 1 | 1 | 0 |
| *Burkholderia vesicularis* | 1 | 1 | 0 |
| *Chryseobacterium meningosepticum* | 1 | 1 | 0 |
| *Moraxella non liquefaciens* | 2 | 2 | 0 |
| *Myroïdes* spp. | 1 | 1 | 0 |
| *Pseudomonas aeruginosa* | 4 | 0 | 4 |
| *Pseudomonas putida* | 1 | 1 | 0 |
| *Pseudomonas stutzeri* | 3 | 3 | 0 |
| *Ralstonia pickettii* | 1 | 1 | 0 |
| *Shewanella algae* | 1 | 1 | 0 |
| *Sphingomonas paucimobilis* | 2 | 2 | 0 |
| *Stenotrophomonas maltophilia* | 2 | 2 | 0 |
| *Vibrio vulnificus* | 1 | 1 | 0 |

### Tableau 3 : Efficacité du substrat β-Alanyl p-nitroanilide

Le résultat est donc très démonstratif puisque l'on distingue aisément la présence de *Pseudomonas aeruginosa* par rapport à d'autres espèces n'ayant pas l'activité enzymatique β-alanine aminopeptidase.

Les substrats ayant comme partie cible de la β-Alanine permettant de mettre en évidence l'activité β-alanine aminopeptidase sont donc particulièrement adaptés à la détection de *Pseudomonas aeruginosa,* et de quelques autres espèces (*Serratia liquefaciens, Pseudomonas marcescens, Pseudomonas mendocina* et *Ochrobactrum anthropi*), dont la fréquence de présence dans un échantillon est bien moindre que pour *Pseudomonas aeruginosa.* De ce fait la détection de la présence de cette activité enzymatique sera le plus souvent en relation avec la présence de *Pseudomonas aeruginosa* dans l'échantillon biologique testé. Il est toutefois possible, comme le démontre le paragraphe qui suit, de différencier *Pseudomonas aeruginosa* d'une de ces autres espèces ayant l'activité β-alanyne aminopeptidase.

### 3°) UTILISATION COMBINEE DE DEUX SUBSTRATS ENZYMATIQUES POUR REVELER DEUX ACTIVITES ENZYMATIQUES DIFFERENTES DONT L'UNE EST L'ACTIVITE ENZYMATIQUE 5-ALANINE AMINOPEPTIDASE :

Cette utilisation de deux substrats pour révéler deux activités enzymatiques différentes est particulièrement intéressante. C'est par exemple le cas des activités β-glucosidase et β-alanine aminopeptidase.

Ainsi l'exemple A du chapitre 2 a montré que les espèces *Pseudomonas aeruginosa* et *Pseudomonas mendocina* possèdent toutes les deux une activité β-alanine aminopeptidase. Il est toutefois possible de différencier ces deux espèces par la recherche de l'activité β-glucosidase qui n'est présente que chez *Pseudomonas mendocina.* Cette enzyme peut par exemple être mise en évidence avec le substrat 6-Chloro-3-indolyl-β-D-glycopyranoside, obtenu chez Biosynth, Staad, Suisse.

La différenciation de ces deux espèces peut être réalisée en milieu gélosé, comme décrit ci-après.

A un milieu Trypticase soja gelosé sont ajoutés :
- 200 mg de 6-Chloro-3-indolyl-β-D-glycopyranoside,
- 50 mg de β-Alanyl-N,N'-diméthyl-p-phénylène-diamine, et
- 15 mg d'Acide 3,5-dihydroxy-2-naphtoïque.

Le milieu est réparti et ensemencé de la façon suivante :
- 10 boîtes avec des cultures pures correspondantes à cinq souches de *Pseudomonas aeruginosa* et cinq souches de *Pseudomonas mendocina,* et
- 2 boîtes avec un mélange de deux souches l'une de *Pseudomonas aeruginosa* et l'autre de *Pseudomonas mendocina.*

Les boîtes de Pétri sont incubées à 35-37°C. Après 18-24 heures d'incubation, la coloration des colonies est observée. Les colonies bleues avec un halo bleu correspondent à *Pseudomonas aeruginosa* et à la présence de l'activité β-alanine aminopeptidase, tandis que les colonies pourpres avec un halo bleu correspondent à *Pseudomonas mendocina* et à la présence des deux activités β-alanine aminopeptidase et β-glucosidase.

Il est ainsi très facile de différencier sur un même milieu un mélange de souches appartenant à ces deux espèces lorsqu'elles sont présentes dans un même échantillon. Cet exemple montre qu'il est possible de combiner plusieurs substrats en relation avec plusieurs réactions enzymatiques dans un même milieu réactionnel, dont une selon la présente invention pour caractériser *Pseudomonas aeruginosa.*

## Revendications

1. Substrat enzymatique constitué d'une partie cible et d'une partie marqueur, l'hydrolyse du substrat permettant la séparation de la partie cible et de la partie marqueur, ladite partie cible caractérisant l'activité enzymatique recherchée, **caractérisé par le fait que** la partie cible permet de révéler une activité enzymatique β-alanine aminopeptidase d'au moins un micro-organisme *Pseudomonas aeruginosa,* et la partie marqueur est une molécule révélant la réaction d'hydrolyse.

2. Substrat, selon la revendication 1, **caractérisé par le fait que** la partie cible est constituée de β-Alanine ou d'un de ses dérivés et la partie marqueur est une molécule chromogène ou fluorescente.

3. Substrat, selon l'une quelconque des revendications 1 ou 2, **caractérisé par le fait qu'**il est de formule :
H₂N-CH₂-CH₂-CO-NH-R,
où H₂N-CH₂-CH₂-CO- est la partie cible et -NH-R est la partie marqueur chromogène ou fluorescente du substrat.

4. Substrat, selon l'une quelconque des revendications 1 à 3, **caractérisé par le fait que** la partie marqueur est constituée de :
- β-Naphtylamine,
- Aminométhylcoumarine, tel que 7-Amino-4-méthyl-coumarine,
- dérivé de l'Aminobenzène, tel que 4-Amino-2,6-dichlorophénol,
- p-Nitroaniline, ou
- 2-Amino-indoxyl ou 3-Amino-indoxyl.

5. Composition permettant la détection d'au moins une souche et/ou une espèce de micro-organismes qui comprend au moins un substrat, selon l'une quelconque des revendications 1 à 4, et un milieu de culture.

6. Composition permettant la détection d'au moins souche et une espèce de micro-organismes ou d'au moins deux souches ou deux espèces de micro-organismes, qui comprend au moins un substrat, selon l'une quelconque des revendications 1 à 4, au moins un autre substrat et un milieu de culture.

7. Composition, selon l'une quelconque des revendications 5 ou 6, **caractérisée par le fait que** le milieu de culture contient un révélateur.

8. Composition, selon l'une quelconque des revendications 5 à 7, **caractérisée par le fait que** le milieu de culture contient :
- du sel de diazonium, lorsque la partie marqueur libérée est la β-Naphtylamine, ou
- de l'Acide 3,5-dihydroxy-2-naphtoïque, lorsque la partie marqueur libérée est un dérivé de l'Aminobenzène, tel que le Dichloro-amino-phénol.

9. Composition, selon l'une quelconque des revendications 5 à 8, **caractérisée par le fait qu'**elle est sous la forme d'un milieu liquide ou d'un milieu gélosé.

10. Procédé de détection de l'espèce bactérienne *Pseudomonas aeruginosa,* consistant :
- à mettre au moins un substrat permettant de détecter l'activité enzymatique β-alanine aminopeptidase en présence d'un échantillon suspecté de contenir au moins un micro-organisme *Pseudomonas aeruginosa,* et
- à observer l'apparition de réactions colorées et/ou fluorescentes.

11. Procédé de détection de l'espèce bactérienne *Pseudomonas aeruginosa,* consistant :
- à mettre au moins un substrat permettant de détecter l'activité enzymatique β-alanine aminopeptidase en présence d'un échantillon suspecté de contenir au moins un micro-organisme *Pseudomonas aeruginosa,*
- à mettre au moins un substrat permettant de détecter une activité enzymatique différente de β-alanine aminopeptidase en présence de l'échantillon suspecté de contenir au moins un autre micro-organisme que *Pseudomonas aeruginosa,* cette autre activité enzymatique permettant de différencier *Pseudomonas aeruginosa* de cet autre micro-organisme, et
- à observer l'apparition de réactions colorées et/ou fluorescentes.

12. Procédé, selon l'une quelconque des revendications 10 ou 11, **caractérisé en ce que** le milieu de culture est gélifié.

13. Procédé, selon la revendication 12, **caractérisé en ce qu'**on ajoute dans le milieu de culture un produit réduisant la migration de la ou des colorations et/ou fluorescences obtenues.
